# EUROPEAN PATENT SPECIFICATION

(11) **EP 1 320 060 B1**
(45) Date of publication and mention of the grant of the patent: **14.12.2011**
(21) Application number: 02028252.1
(22) Date of filing: 16.12.2002
(51) Int. Cl.: B01L 99/00

(54) **Printing device for personal medical monitors**
Drucker für persönnliches medizinisches Messgerät
Imprimante pour moniteur medical personnel

(30) Priority: 17.12.2001 US 339809 P; 11.12.2002 US 316679 P
(43) Date of publication of application: 18.06.2003
(73) Proprietor: Becton Dickinson and Company, Franklin Lakes, New Jersey 07417-1880 (US)
(72) Inventor: Gordon, Tim H., River Vale, New Jersey 07675 (US); Gisler, Scott W., Pine Brook, New Jersey 07058 (US); Puma, Michael, Midland Park, New Jersey 07432 (US); Yao, Raymond L., Hoboken, New Jersey 07030 (US); Cassigneul, Pierre, Flemington, New Jersey 08822 (US)
(74) Representative: von Kreisler Selting Werner

(56) References cited:
- WO-A-00/28460
- WO-A-94/19684
- DE-A- 19 546 535
- US-A- 3 502 438

## Description

### Field of the Invention

The present invention relates to personal medical monitors, such as blood glucose meters (BGMs). In particular, the present invention relates to a printing device for retrieving and analyzing data from a personal medical monitor, such as a body fluid chemistry analyzer, and for formatting the data and printing a result based on the data. The output can include a log of data entries from the personal medical monitor, and can also include graphical representations derived from the data, such as pie charts, trends, histograms, and the like. Furthermore, a printing device according to the present invention can apply therapy rules, and include therapy recommendations in a printout based on analysis of existing data, in order to provide therapy guidance to both a healthcare professional and a patient.

### Background of the Invention

The value of a medical device that can generate and store clinical data is realized in the subsequent utilization of that data to affect clinical outcomes. One example is the utilization of self monitored blood glucose (SMBG) data for diabetes management. Numerous factors presently constrain more effective use of SMBG data generated by blood glucose meters. Both patients and health care providers are constrained by the limitations of current hardware, data analysis and presentation, time, training, and application of therapy principles, to the valuable data generated by patients every day.

Current SMBG data utilization is accomplished with the use of a meter's display, review of a patient's hand generated logbook, or by connecting the meter and downloading its data into a PC based software program. Unfortunately, each of these methods has inherent shortfalls. Data utilization based on the meter display and patient logbook is better than not using the data at all, but is limited by the clinical knowledge and analytical capabilities of the user. Additionally, a hand generated logbook is prone to frequent errors, thus the data lacks integrity.

Data utilization based on current PC download systems enhance the user's ability to analyze the data to some extent. However, current PC systems fall short in at least two respects. First, current systems present ease of use issues. The user must be familiar with connecting and configuring the system, as well as accessing the system and presenting data in a meaningful and useful format. Second, current systems provide only limited direction on how to apply the analyzed data to enhance clinical decisions.

WO 94/19684 discloses a portable immediate response medical analyzer comprising a single use disposable cartridge adapted to interface with an associated portable electroanalytical instrument used in making electrochemical determinations. The cartridge further includes a sample chamber for receiving a sample of the serum of interest, an array of electrochemical sensors and in situ calibration media. The portable instrument has a receptacle for receiving the disposable cartridge and an electrical interface for connecting with electrical interface terminals of the cartridge. After connecting the cartridge to the portable electroanalytical instrument, the cartridge automatically self-calibrates relative to the instrument. Afterwards, a sample of the serum of interest is transferred into the sample chamber of the disposable cartridge and the portable electroanalytical instrument performs an immediate evaluation of the sample.

### Summary of the Invention

The device of the invention is defined by claim 1. The method of the invention is defined by claim 10.

The present invention overcomes the above disadvantages and other advantages are realized by providing a system according to the present invention. In one embodiment of the present invention, an output device, such as a printer, is provided with an interface for receiving a body fluid chemistry analyzer, such as a blood glucose monitor. Data is transferred from the blood glucose monitor to the output device through the interface. The data is analyzed and formatted into graphical representations, which are printed for evaluation by both the patient and a health care provider. In another embodiment, therapy rules are applied to the data by the output device, and therapy recommendations are included in the printout by the output device based on the data to provide therapy guidance to both a health care provider and a patient.

### Brief Description of the Drawings

The invention will be further understood with reference to the attached drawing figures, in which:

Figure 1 illustrates a first embodiment of an exemplary system according to the present invention;

Figure 2 illustrates a second embodiment of an exemplary system according to the present invention;

Figure 3 illustrates a third embodiment of an exemplary system according to the present invention;

Figure 4 is a block diagram of an exemplary embodiment of a system according to the present invention;

Figure 5 is a flowchart illustrating an exemplary method according to an embodiment of the present invention;

Figures 6A-6C illustrate alternate embodiments of a printing device according to the invention; and

Figure 7 is a flow chart illustrating the functionality of a processor adapted to control a printing device according to an embodiment of the present invention.

Throughout the drawing figures, it will be understood that like numerals refer to like structures or steps.

### Detailed Description of the Preferred Embodiments

The following description of the presently contemplated best mode of practicing the invention and preferred embodiments thereof is not to be taken in a limiting sense, but is provided merely for the purpose of describing the general principles of the invention.

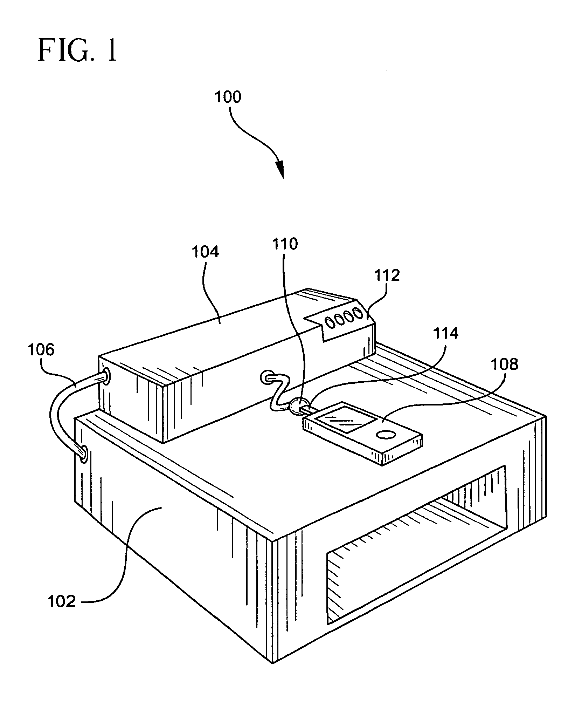

Figure 1 illustrates a first embodiment of a system 100 according to the present invention. The system comprises a printer 102 connected to an electronics package 104 by a printer connector cable 106. The system further comprises a personal medical monitor 108, such as a blood glucose meter, connected to the electronics package 104 by an interface cable 110. The electronics package 104 has status indicators 112, which are LEDs for instance, which are adapted to provide visual feedback on the status of the system. The status indicators 112 indicate, among other things, whether the system 100 is in an idle ready state, whether the system 100 is connected to a personal medical monitor 108, whether the system 100 is presently printing, or whether the printer 102 needs maintenance. The personal medical monitor 108 has an interface connector 114 which is adapted to be connected to the interface cable 110 in order to facilitate data transfer between the personal medical monitor and the electronics package 104.

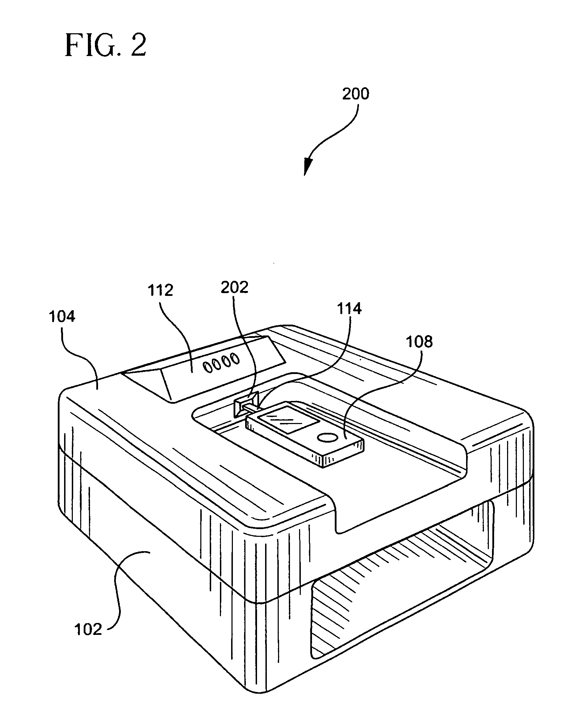

Figure 2 illustrates a second embodiment of a system 200 according to the present invention. In this embodiment, the electronics package 104 is integrated into the body of the printer 102. Furthermore, the interface cable 110 is replaced by an interface 202 which is also integrated into the body of the printer 102. The interface or port 202 (which can consist of a plug-and-socket arrangement, for example) facilitates data transfer from the personal medical monitor 108 through the interface connector 114 of the personal medical monitor 108 when the personal medical monitor 108 is connected to the interface 202.

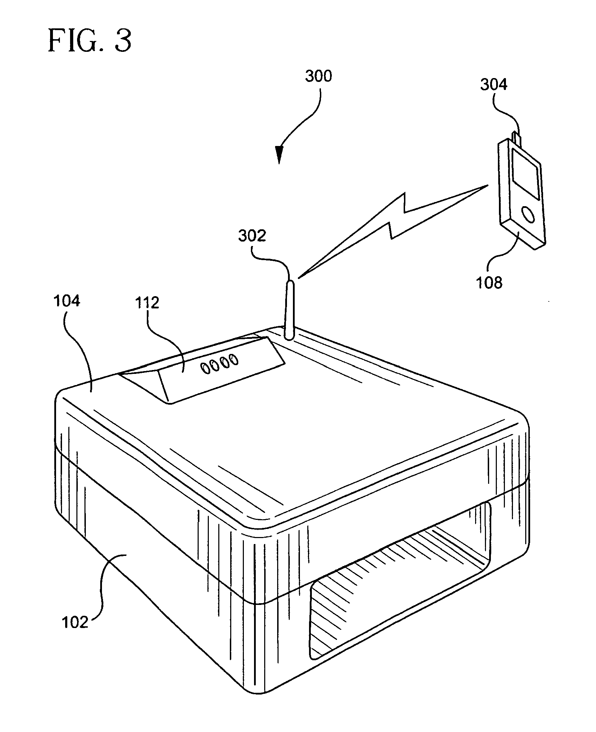

Figure 3 illustrates a third embodiment of a system 300 according to the present invention. In this embodiment, the printer 102 and personal medical monitor 108 are provided with antennas 302, 204 to facilitate wireless data transfer between the personal medical monitor 108 and the electronics package 104 of the printer 102.

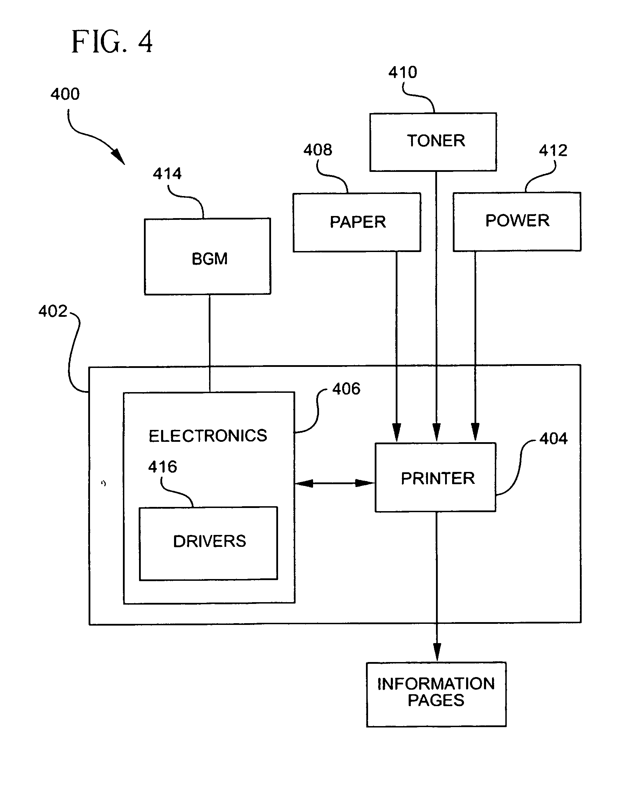

Figure 4 is a block diagram of a system 400 according to the present invention. The output device 402 preferably comprises a printer 404 and electronics 406. The printer 404 preferably receives paper 408, toner or ink 410 and power 412. The paper 480 can preferably be plain paper in a standard format, such as 8.5" x 11", or A4 sized paper. The paper also may be printed letterhead paper, or have other pre-printed information on it, such as promotional or instructional information, which is not dependent on the data. Of course, one of skill in the art will readily appreciate that the invention is not limited to any particular form factor of paper, and furthermore, the invention is contemplated to be useful in connection with a wide variety of printing and display technologies. The printer 404 is preferably a laser printer, and uses toner 410 to print pages. However, the invention is not limited to laser printers, and any printer such as an inkjet printer is considered to be within the scope of the invention. The printer 404 preferably uses standard 120V AC power 412, such as is available in most wall outlets. However, other power sources may be used, as would be convenient and appropriate in a given situation, and any appropriate power supply is considered to be within the scope of the invention. Furthermore, the output device 402 could be powered by batteries (not shown) in lieu of an outside power source, or as a back-up power source.

The electronics 406 perform a number of tasks. Among them, the electronics 406 manage the transfer of data from a blood glucose monitor 414 when a blood glucose monitor 414 is connected to the device 402. Preferably, the electronics are adapted to recognize when a blood glucose monitor 414 has been connected to the output device 402, and begin effectuating a data transfer without any intervention by the user. In the preferred embodiment, the output device 402 has an interface 202 incorporated therein, comprising a physical interface for receiving data from the blood glucose monitor 414. The blood glucose monitor 414 has a corresponding interface 114 adapted to fit the interface 202 of the output device 402, and the user simply inserts the blood glucose monitor 414 into the interface of the output device 402 to begin a data transfer and print operation.

In a preferred embodiment of the invention, the printer 404 has any necessary driver software and configuration software required to interface with a bodily fluid chemistry analyzer pre-installed prior to delivery to an intended user, such as a health care professional's office. This is referred to herein as being pre-configured. Thus, in this embodiment, once the printer 404 is delivered to the user's location, all that is required to use the device is to power it up and connect a blood fluid chemistry analyzer. The driver and configuration software 416 are preferably contained in the electronics 406 of the printer as shown in Figure 4. Alternatively, they could be stored on a computer readable media, which is adapted to be accessed by the electronics 406.

In another embodiment, the output device 402 comprises a wireless transceiver (not shown) for communicating with a blood glucose monitor 414 having wireless communication ability. The output device 402 is outfitted with a Bluetooth or WiFi (IEEE 802.11b protocol) wireless device, or the like, which provides an alert to the electronics 406 when a blood glucose monitor 414 is within range for wireless communication. Of course those of skill in the art will readily appreciate that the invention is not limited to any particular wireless technology, and could include, among other technologies, any custom or proprietary magnetic, RF, or infrared wireless communication protocol. The electronics 406 then establish a communication link with the blood glucose monitor 414, which allows data to be transferred to the output device 402 by wireless means. In one embodiment the blood glucose monitor 414 is adapted to provide an alert to the user which indicates that a connection with the output device 402 is available. By pressing a button on the blood glucose monitor 414, or otherwise indicating a desire to transfer data to the output device, a data transfer from the blood glucose device 414 to the output device 402 is caused.

The electronics 406 are further adapted to format printed pages based on the received SMBG data, generate graphical representations of data, such as pie charts, histograms, and the like, and to further format any information which is to be printed. The electronics 406 further drive the printer 404. Finally, the electronics 406 manage any other interfaces which may be provided with the system 400, such as a link to a PC or network for data storage or retrieval, configuration, software upgrades, and the like.

In one embodiment of the invention, the electronics 406 are further provided with logic to analyze the data received from a blood glucose monitor 414 and to apply therapy rules to the data. In this manner, the device is capable of providing therapy recommendations based on an analysis of the SMBG data, along with other printed matter such as graphical displays of the data, in order to provide therapy guidance to the patient and the healthcare provider.

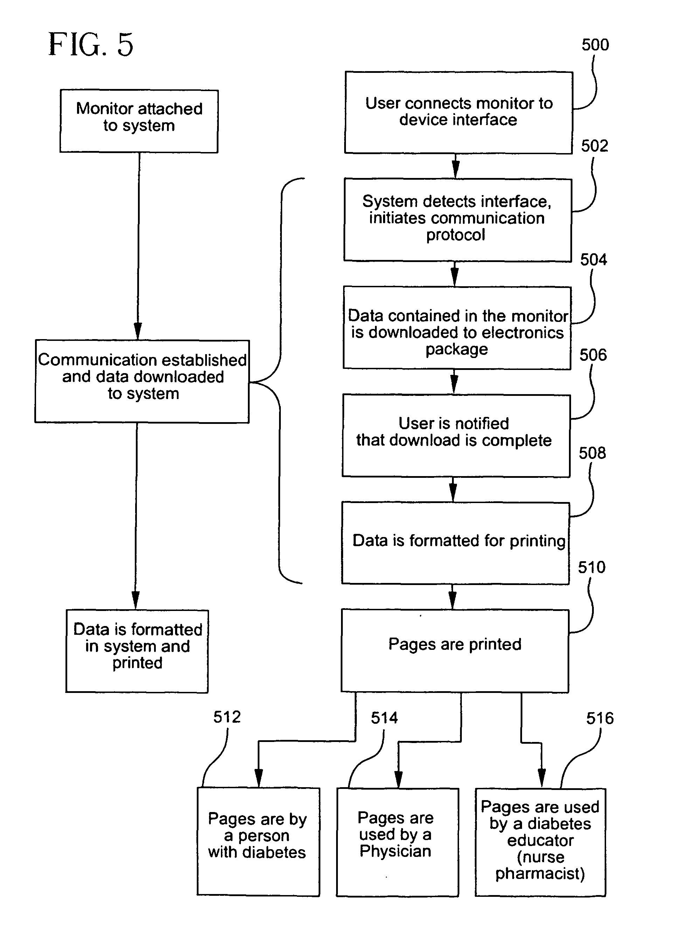

Figure 5 is a flow chart illustrating the functionality of a system according to an embodiment of the present invention. The device 402 is preferably activated by connecting a monitor 414 to the device 402 at step 500. According to the preferred embodiment of the invention, this is the only action which must be taken by the user to produce an output. It will be recognized by those of skill in the art that a physical connection is not required, and data transfer may be accomplished by wireless means, such as for instance by the Bluetooth or 802.11b wireless protocols or through an infrared data port, for example. In a wireless embodiment, rather than physically connecting the monitor to a printing device, the user may only be required to place the monitor in proximity to a printing device. The monitor is adapted to recognize a suitable printer within its proximity and alert the user to the established communication between monitor and printer. In this case, the user simply indicates the desire for a data transfer to occur, as for example, by pressing an appropriate button provided on the monitor for this purpose.

Once the monitor 414 is connected, as described in connection with Figure 2, the device 402 detects the monitor 414 and initiates a communications protocol with the monitor, at step 502, in order to download data from the monitor 414 to the device 402. At step 504, the data contained in the monitor 414 is downloaded to the electronics 406 of the device 402 to be analyzed and processed. The user is notified once the data transfer has been completed at step 506.

At step 508, the data which was received from the monitor 414 is analyzed and processed. The data is formatted for printing. Preferably, the formatting includes generating graphical displays of the data, such as predefined histograms, line charts, and any other graphical displays which might be helpful to the healthcare professional and the patient. In addition, during data processing, a set of therapy rules can be applied to the data, resulting in the output of a therapy recommendation. As an example, the data downloaded from the monitor 414 could include information indicating that the patient is receiving insulin twice per day. The data could further reveal that the patient experiences hypergycemia in the early afternoons (typically after lunch). Based on these factors, the printing device could apply therapy rules and generate a recommendation to start the patient on a three dose per day insulin program. In this manner a system according to the present invention can provide therapy guidance to the healthcare professional for the benefit of the patient. Of course the therapy rules applied by the system are limited only by the sophistication and processing power of the hardware, along with the level of detail of the software utilized by the system. A wide range of potential therapy rules are contemplated to be within the scope of the invention.

At step 510, the printer outputs printed pages containing the formatted data, such as graphical displays, logbook data, and therapy recommendations, as described above. Finally, at steps 512, 514, and 516, the printout can be used by a patient, a health care professional, or an educator. It should be understood that a paper printout is described in connection with the preferred embodiment of the invention, but the invention is not limited to a paper printout. Accordingly, an output to a CRT or LCD display, or the like, are contemplated to be within the scope of the invention.

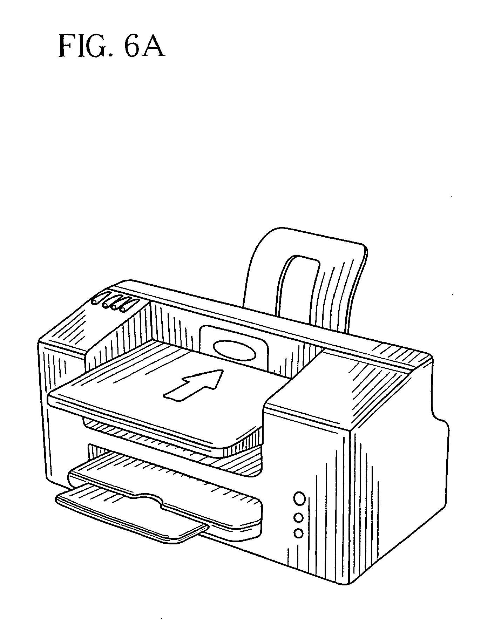

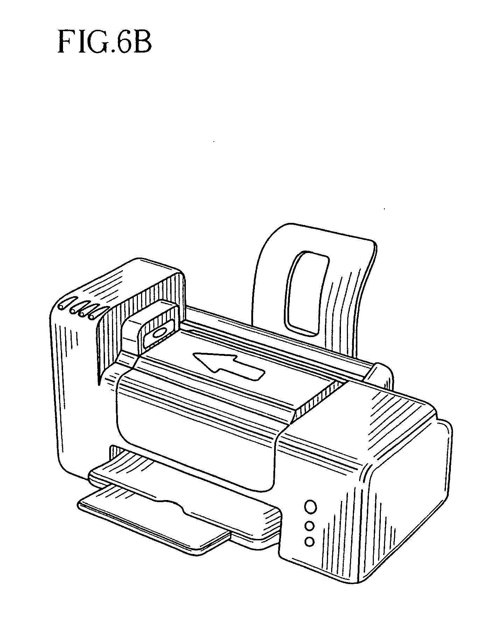

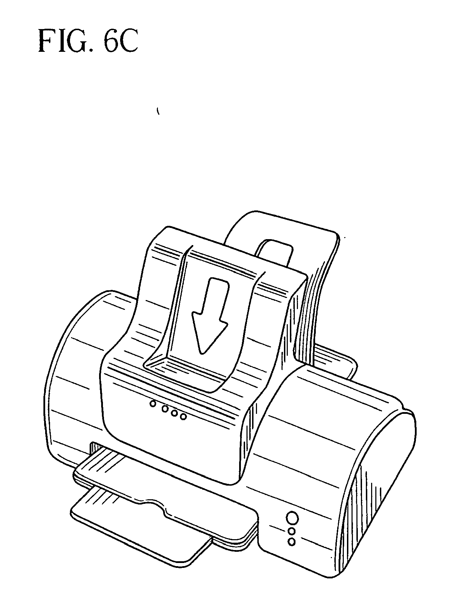

Figures 6A-6C illustrate alternate embodiments of a printing device. Figure 6A illustrates an inkjet style printer with a front oriented connector for the body fluid chemistry analyzer. Figure 6B illustrates another embodiment of the inkjet style printer with a side oriented connector for the body fluid chemistry analyzer. Figure 6C illustrates an embodiment of the inkjet style printer with a top oriented connector for the body fluid chemistry analyzer.

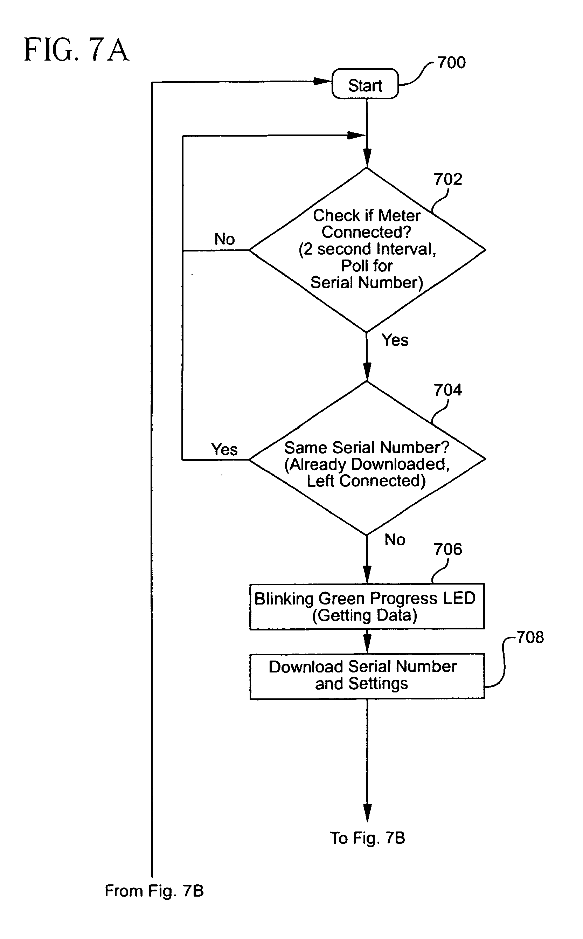

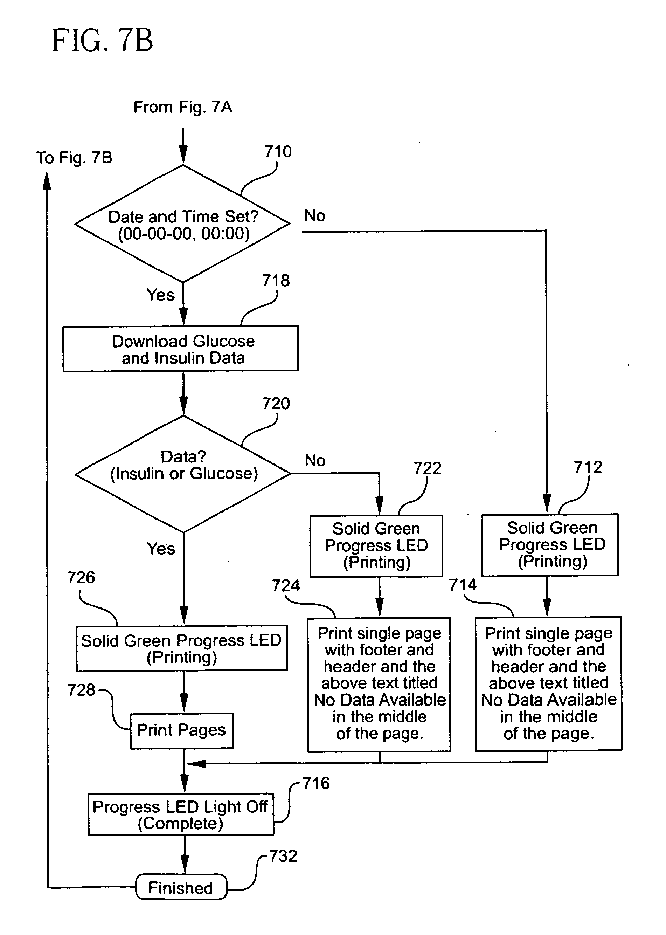

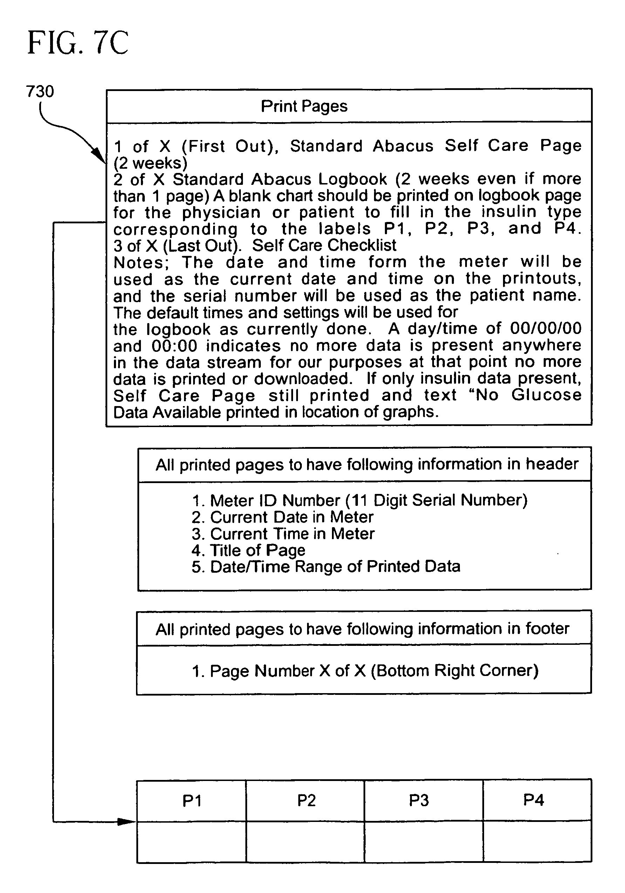

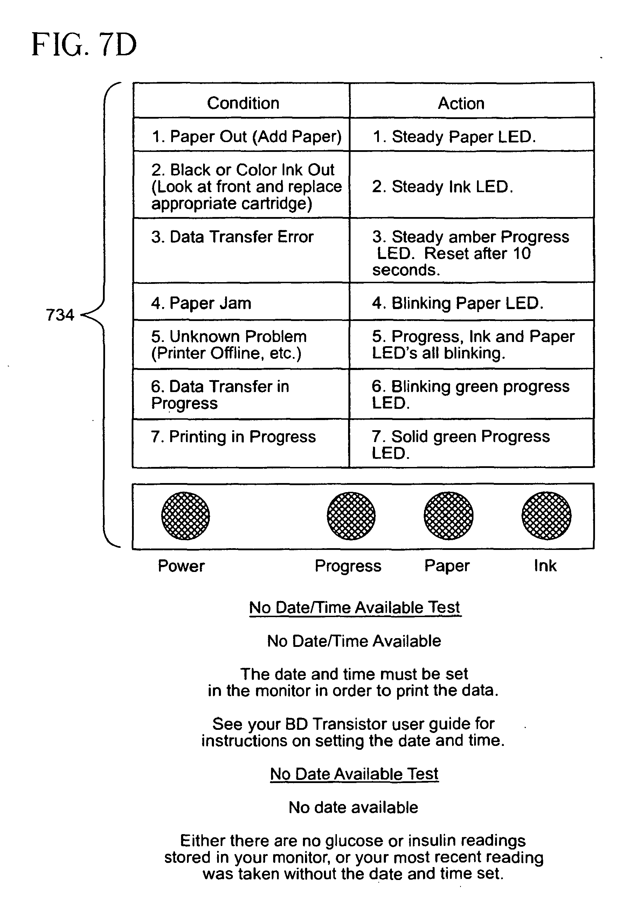

Figure 7 is a more detailed flowchart illustrating the program flow of the processor controlling a printing device according to an embodiment of the invention. Program flow starts at step 700. At 702, the printer determines whether a meter is connected. If a meter is connected, the program determines the serial number of the meter, and whether it is a new serial number at step 704. If it is a new serial number, program flow continues to step 706. While data is being retrieved from the meter, an LED indicator is made to blink (step 706). The meter's serial number and settings are downloaded at step 708.

At step 710, the program determines whether the meter's date and time have been set. If the time and date have not been set, program flow continues to 712, where an LED is solidly lit while a page is printed at step 714. The page indicates that the time and date of the meter have not been set. Program flow then continues to step 716 to be described below. If during step 710, the program determines that the data and time of the meter have been set, program flow continues to step 718, where glucose and insulin data are downloaded. Next, at step 720, the program determines whether any glucose or insulin data was downloaded. If no data was downloaded during step 718, the program flow continues to step 722, in which an LED is solidly lit, while a page is printed at step 724. The printed page indicates that no data was available.

If during step 720, it is determined that glucose and insulin data was downloaded, program flow continues to step 726, in which an LED is solidly lit, while printed pages are produced at step 728. The format and content of printed pages is described generally at 730. Status and error conditions are also preferably indicated by controlling a set of LED's, as indicated generally at 734. At step 716, when all pages to be printed have completed printing, the progress LED is turned off. Program flow concludes at step 732, at which point program flow preferably loops back to the start, at step 700.

While the invention herein disclosed has been described by means of specific embodiments and applications thereof, numerous modifications and variations can be made thereto by those skilled in the art without departing from the scope of the invention.

## Claims

1. A printing device (100, 200, 300, 400) for use with a body fluid chemistry analyzer, comprising:
a communication port (110, 202, 302) adapted for communication with a body fluid chemistry analyzer (108, 414),
a printer (102, 404) and
a processor or electronics (104, 406), wherein said processor is adapted to, in response to a single action by a user, retrieve body fluid chemistry data from said body fluid chemistry analyzer (108, 414), format said data, and control said printer to print said formatted data.

2. The printing device (100, 200, 300, 400) of claim 1, wherein said printing device has a set of drivers for interfacing with said body fluid chemistry analyzer (108, 414) pre-installed prior to delivery to an intended user.

3. The printing device of claim 1, wherein said single action comprises connecting said body fluid chemistry analyzer (108, 414) to said communication port (110, 202, 302).

4. The printing device (100, 200, 300, 400) of one of claims 1 - 3, wherein said body fluid chemistry analyzer (108, 414) comprises a blood glucose monitor.

5. The printing device (100, 200, 300, 400) of one of claims 1 - 4, wherein said communication port (110, 202, 302) is a wireless communication port, said printing device further comprising a monitoring circuit adapted to poll for an available wireless communication device, to establish a communication link with said available wireless communication device, and to provide an alert to said user indicating the presence of said wireless communication device, and wherein said single action comprises responding to said alert.

6. The printing device (100, 200, 300, 400) of claim 5, wherein said wireless communication port comprises an infrared port.

7. The printing device (100, 200, 300, 400) of claim 5, wherein said wireless communication port comprises an IEEE 802.11 port.

8. The printing device (100, 200, 300, 400) of claim 5, wherein said wireless communication port comprises a Bluetooth port.

9. The printing device (100, 200, 300, 400) of one of claims 1 - 8, wherein said processor is further adapted to generate charts from said data, and to print said charts in response to said single action.

10. A method of retrieving and printing stored blood chemistry data using the device of one of claims 1-9, comprising the steps of:
in response to a single action by a user, retrieving body fluid chemistry analyzer data from a body fluid chemistry analyzer (108, 414) through a communication port (110, 202, 302) of a printing device (100, 200, 300, 400),
formatting said data, and
printing said formatted data.

## Patentansprüche

1. Druckvorrichtung (100, 200, 300, 400) zur Verwendung mit einer chemischen Körperflüssigkeit-Analysevorrichtung, mit:
einem Kommunikationsport (110, 202, 302), der zur Kommunikation mit einer chemischen Körperflüssigkeit-Analysevorrichtung (108, 414) geeignet ist,
einem Drucker (102, 404) und
einem Prozessor oder einer Elektronik (104, 406), wobei der Prozessor geeignet ist, in Reaktion auf eine einzelne Aktion eines Benutzers, chemische Körperflüssigkeitsdaten von der chemischen Körperflüssigkeit-Analysevorrichtung (108, 414) abzurufen, diese Daten zu formatieren und den Drucker zum Ausdrucken der formatierten Daten anzusteuern.

2. Druckvorrichtung (100, 200, 300, 400) nach Anspruch 1, bei welcher die Druckvorrichtung einen vor der Auslieferung an den vorgesehenen Benutzer vorinstallierten Satz Treiber zur Anbindung an die chemische Körperflüssigkeit-Analysevorrichtung (108, 414) aufweist.

3. Druckvorrichtung nach Anspruch 1, bei welcher die einzelne Aktion das Anschließen der chemischen Körperflüssigkeit-Analysevorrichtung (108, 414) an den Kommunikationsport (110, 202, 302) umfasst.

4. Druckvorrichtung (100, 200, 300, 400) nach einem der Ansprüche 1-3, bei welcher die chemische Körperflüssigkeit-Analysevorrichtung (108, 414) ein Blutzucker-Messgerät aufweist.

5. Druckvorrichtung (100, 200, 300, 400) nach einem der Ansprüche 1-4, bei welcher der Kommunikationsport (110, 202, 302) ein Drahtlos-Kommunikationsport ist, wobei die Druckvorrichtung ferner eine Überwachungsschaltung aufweist, die geeignet ist, nach einer verfügbaren Drahtlos-Kommunikationsvorrichtung zu suchen, um eine Kommunikationsverbindung mit der verfügbaren Drahtlos-Kommunikationsvorrichtung herzustellen, und um einen Alarm an den Benutzer auszugeben, welcher das Vorhandensein der Drahtlos-Kommunikationsvorrichtung anzeigt, und wobei die einzelne Aktion das Reagieren auf den Alarm umfasst.

6. Druckvorrichtung (100, 200, 300, 400) nach Anspruch 5, bei welcher der Drahtlos-Kommunikationsport einen Infrarotport aufweist.

7. Druckvorrichtung (100, 200, 300, 400) nach Anspruch 5, bei welcher der Drahtlos-Kommunikationsport einen IEEE 802.11 Port aufweist.

8. Druckvorrichtung (100, 200, 300, 400) nach Anspruch 5, bei welcher der Drahtlos-Kommunikationsport einen Bluetooth-Port aufweist.

9. Druckvorrichtung (100, 200, 300, 400) nach einem der Ansprüche 1-8, bei welcher der Prozessor ferner geeignet ist, aus den Daten Diagramme zu erzeugen und diese Diagramme in Reaktion auf die einzelne Aktion zu drucken.

10. Verfahren zum Abrufen und Drucken von gespeicherten chemischen Blutdaten unter Verwendung der Vorrichtung nach einem der Ansprüche 1-9, mit den folgenden Schritten:
in Reaktion auf eine einzelne Aktion eines Benutzers, Abrufen von chemischen Körperflüssigkeit-Analysevorrichtungsdaten aus einer chemischen Körperflüssigkeit-Analysevorrichtung (108, 414) über einen Kommunikationsport (110, 202, 302) einer Druckvorrichtung (100, 200, 300, 400), Formatieren der Daten, und Drucken der formatierten Daten.

## Revendications

1. Imprimante (100, 200, 300, 400) destinée à être utilisée avec un analyseur chimique de fluides corporels, comprenant:
un port de communication (110, 202, 302) apte à communiquer avec un analyseur chimique de fluides corporels (108, 414),
une imprimante (102, 404) et
un processeur ou des moyens électroniques (104, 406), ledit processeur étant apte à retrouver, en réaction sur une seule action de l'utilisateur, des données chimiques de fluides corporels dans ledit analyseur chimique de fluides corporels (108, 414), à formater les données et à commander l'imprimante pour qu'elle imprime les données formatées.

2. Imprimante (100, 200, 300, 400) selon la revendication 1, dans laquelle ladite imprimante contient un groupe de pilotes pour permettre l'interfaçage avec ledit analyseur chimique de fluides corporels (108, 414), lesdits pilotes étant préinstallés avant livraison à un utilisateur spécifique.

3. Imprimante selon la revendication 1, dans laquelle ladite seule action comprend la connexion dudit analyseur chimique de fluides corporels (108, 414) audit port de communication (110, 202, 302).

4. Imprimante (100, 200, 300, 400) selon l'une des revendications 1-3, dans laquelle ledit analyseur chimique de fluides corporels (108, 414) comprend un moniteur de mesure de la glycémie.

5. Imprimante (100, 200, 300, 400) selon l'une des revendications 1-4, dans laquelle ledit port de communication (110, 202, 302) est un port de communication sans fil, ladite imprimante comprenant en outre un circuit moniteur apte à chercher un dispositif de communication sans fil disponible, afin d'établir un lien de communication avec ledit dispositif de communication sans fil disponible, et à fournir un alarme à l'utilisateur indiquant la présence d'un dispositif de communication sans fil, et ladite seule action comprend une réaction sur cet alarme.

6. Imprimante (100, 200, 300, 400) selon la revendication 5, dans laquelle ledit port de communication sans fil comprend un port à infrarouge.

7. Imprimante (100, 200, 300, 400) selon la revendication 5, dans laquelle ledit port de communication sans fil comprend un port du type IEEE 802.11.

8. Imprimante (100, 200, 300, 400) selon la revendication 5, dans laquelle ledit port de communication sans fil comprend un port Bluetooth.

9. Imprimante (100, 200, 300, 400) selon l'une des revendications 1-8, dans laquelle ledit processeur est apte en outre à générer des diagrammes à partir desdites données et à imprimer les diagrammes en réaction sur ladite seule action.

10. Procédé pour retrouver et imprimer des données chimiques de sang enregistrées, utilisant l'imprimante selon l'une des revendications 1-9, ledit procédé comprenant les étapes suivantes:
en réaction sur une seule action d'un utilisateur, retrouver des données de l'analyseur chimique de fluides corporels dans un analyseur chimique de fluides corporels (108, 414) à travers un port de communication (110, 202, 302) d'une imprimante (100, 200, 300, 400),
formater les données, et
imprimer les données formatées.
